# EUROPEAN PATENT SPECIFICATION

(11) **EP 0 569 241 B1**
(45) Date of publication and mention of the grant of the patent: **22.12.1999**
(21) Application number: 93303521.4
(22) Date of filing: 06.05.1993
(51) Int. Cl.: A61M 13/00

(54) **Apparatus for insufflation with gas**
Vorrichtung zur Insufflation mit Gas
Dispositif d'insufflation avec gas

(30) Priority: 07.05.1992 AU 231392; 07.05.1992 AU 231292; 17.09.1992 AU 474892
(43) Date of publication of application: 10.11.1993
(73) Proprietor: William A. Cook Australia Pty. Ltd., Eight Miles Plains, Brisbane, Queensland 4113 (AU)
(72) Inventor: Ward, Andrew W., Ashgrove, Q 4060 (AU); O'Brien, Sean, Redland Bay, Q 4165 (AU); Reeves, Geoff M., Burbank, Q 4156 (AU)
(74) Representative: Johnston, Kenneth Graham

(56) References cited:
- EP-A- 0 082 041
- EP-A- 0 169 972
- EP-A- 0 446 715
- DE-C- 3 611 018
- US-A- 4 681 099
- US-A- 5 006 109
- US-A- 5 013 294

## Description

### Technical Field

This invention relates generally to an apparatus for the insufflation of gas into a human or animal body and, in particular, to an improved apparatus of insufflation.

### Background of the Invention

Minimally invasive surgical procedures such as laparoscopy and hysteroscopy require the insufflation of gas into a body cavity. Known insufflation apparatuses pass gas from the pressurized reservoir through the gas line and an insufflation or Veress needle into the body cavity. The gas flow rate is measured by the meter in the gas line and controlled by the gas regulator. The pressure in the body cavity is measured by the pressure meter within the insufflation apparatus and gas is delivered at a selected rate until the desired pressure is reached within the body cavity. Commercially available, electronic insufflation apparatuses typically provide gas flow rates in the range of 6 to 10 liters per minute. However, as surgical procedures utilizing insufflation become more sophisticated and complicated, there is a demand for delivery of gas at higher flow rates. The greater the number of access sheaths used during a procedure, for example, the more rapidly gas escapes from an insufflated body cavity, Furthermore, in surgical procedures utilizing lasers, the smoke generated by the lasers temporarily obscures vision at the surgical site. The smoke can be readily dissipated by introducing a leak into the body cavity, but high gas flow rates are required to maintain pressure in the insufflated body cavity.

A problem with the use of known insufflation apparatuses at higher flow rates is that the amount of gas introduced into the body cavity must be carefully controlled to avoid over-inflation of the body cavity. Over-inflation of the body cavity can induce injury to hollow vessels and organs of the body due to compression and reduced blood flow. Over-inflation also can compromise cardiovascular function by impairing venous return to the heart. Another problem with the use of known apparatuses at higher flow rates is the likelihood of increased gas absorption from the insufflated body cavity into the blood stream or subcutaneous tissues of the body. When insufflating a body cavity such as the peritoneum with CO₂, for example, the absorption of CO₂ can result in any of a number of complications, including subcutaneous emphysema, hemodynamic decompensation, compromised cardiovascular performance, hypercarbia, acidosis, hypoxemia, hypotension, increased heart rate, cardiac arrythmia, and compromised cardiovascular performance. Moreover, the risk of cardiac arrhythmia is increased during a procedure utilizing catecholamines and anaesthetic agents in conjunction with CO₂, insufflation.

Yet another problem with the use of known insufflation apparatuses at higher gas flow rates is that all known insufflation gas is derived from a pressurized gas reservoir. As the gas passes from the pressurized reservoir through the insufflation needle, the components or the insufflation apparatus exhibit a cooling effect. Higher gas flow rates increase the cooling effect. As a result, there is condensation of water within the insufflation apparatus. Furthermore, a significant cooling effect within the body cavity of the patient can occur.

Still yet another problem with the use of known insufflation apparatuses at higher gas flow rates is that all known insufflation apparatuses utilize a single pressurized gas reservoir for the supply of gas. The size of the gas reservoir varies, but is generally of a size sufficient for supplying gas for several relatively simple, uncomplicated surgical procedures. However, larger volumes of gas are needed for surgical procedures that are more sophisticated and complicated, as previously suggested. As a result, there is an increasing trend to attach larger pressurized gas reservoirs to existing insufflation apparatuses. A problem with the attachment of larger reservoirs to existing apparatuses is that a large gas reservoir makes the apparatus bulky, cumbersome, and less mobile. Another problem with the approach of attaching larger reservoirs is that the storage and handling of large pressurized gas reservoirs present an increased safety risk.

EPA- 169972 discloses a gas insufflation system as described in the preamble of claim 1.

According to the present invention, there is provided an insufflation system as defined in claim 1.

A technical advance is achieved in an illustrative gas insufflation system as claimed in claim 1. The insufflation system advantageously includes an electronic control circuit responsive to gas pressure and flow rate in a gas supply line for controlling a gas supply system that supplies insufflation gas to the line.

The insufflation system further comprises a patient monitoring system which is connected to the electronic control circuit. The monitoring system is responsive to at least one of a plurality of patient physiological parameters for signaling the electronic control circuit. When the patient physiological parameter reaches a predetermined level, the electronic control circuit controls the gas pressure and flow rate in the line in response to the said at least on patient physiological parameter.

The gas supply system includes a first pressurized gas storage reservoir, pressure and flow rate measuring transducers coupled to the electronic control circuit, and a gas regulator all connected to the gas line. The gas supply system further includes an electronically operated valve connected to the gas line and responsive to the electronic control circuit for controlling the flow of gas in the line.

The gas supply system further comprises a second gas reservoir and an electronically controlled valve coupled to the first and second gas reservoirs and responsive to the electronic control circuit for selectively connecting the gas line to the first and second gas reservoirs.

To reduce cooling of the gas during expansion from the pressurized gas reservoirs, a heating element and thermal insulation is disposed proximate the gas line and at least one of a pressure reducer and a gas regulator.

### Brief Description of the Drawing

FIG. 1 depicts a schematic block diagram of an insufflation system of the present invention with a patient monitoring system attached thereto;
FIG. 2 depicts a schematic block diagram of an enhancement to the insufflation system of FIG. 1 with insulating and heating elements; and
FIG. 3 depicts a schematic block diagram of another aspect of the insufflation system of FIG. 1 with a gas supply system that accommodates two gas reservoirs.

### Detailed Description

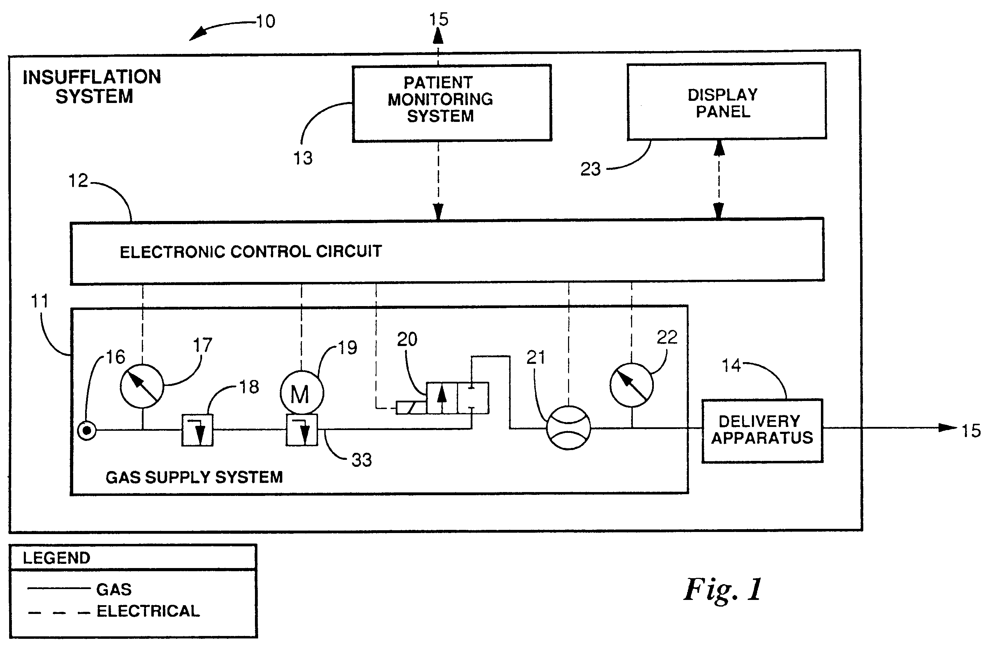

FIG. 1 depicts a schematic block diagram of an illustrative insufflation system 10 of the present invention with a patient monitoring system 13 attached thereto. Insufflation system 10 includes gas supply system 11, which passes gas from a pressurized reservoir 16 through gas line 33 and into a body cavity via insufflation delivery apparatus 14 such as a Veress needle positioned in communication with the body cavity of patient 15. Insufflation system 10 further includes a well-known electronic control circuit 12 for controlling the flow of gag and a well-known patient monitoring system 13, which monitors a physiological patient parameter and supplies information from patient 15 to the electronic circuit. Alternatively, electronic circuit 12 is a microprocessor. Furthermore, patient monitoring system 13 can be integrated into the same housing as electronic circuit 12, rather than being connected to the electronic circuit via an external connector as illustrated.

Patient monitoring system 13 preferably measures end-tidal CO₂ (commonly referred to as capnograph). When end-tidal CO₂ levels indicate that significant absorption of CO₂ has occurred, the electronic control circuit reduces or stops delivery of CO₂ gas to the body cavity and/or notifies the surgeon that the patient is at risk. Alternatively, the patient monitoring system is a pulse oximeter, an ECG, or a multifunction patient monitoring system formed from a combination of these patent monitoring elements. When the patient monitoring system includes a pulse oximeter, reduced hemoglobin saturation is communicated to the electronic control circuit for reducing or stopping the delivery of CO₂ gas to the body cavity and/or notifying the surgeon that the patient is at risk. When the patient monitoring system includes an ECG, cardiovascular complications or irregularities such as cardiac arrhythmias or increased heart rate are communicated to the electronic control circuit, again, for reducing or stopping the delivery of CO₂ gas to the body cavity and/or notifying the surgeon that the patient is at risk.

Gas supply system 11 includes pressurized gas storage reservoir 16 which is connected via gas line 33 to well-known pressure reducer 18. The pressure reducer reduces the high pressure in the gas reservoir down to, for example, 40-50 kPa. Pressure measuring means such as well-known pressure transducer 17 is positioned in gas line 33 between the gas reservoir and the pressure reducer for supplying information to electronic control circuit 12. The electronic control circuit controls gas flow rate by signalling adjustable gas regulation means 19 such as a motor operated gas regulator or an electromagnetically operated regulator for regulating either pressure or flow. Alternatively, gas regulation means 19 is formed from two or more electronically operated valves such as solenoid valves for directing flow through various flow limiters. Yet another alternate construction of regulation means 19 is a branch pressure feedback gas line to operate a pressure drip operated flow regulator. The adjustable gas regulator is positioned downstream of pressure reducer 18.

Electronic circuit 12 also controls an electronically operated valve 20 such as a solenoid valve for blocking gas line 33 and stopping gas flow. Flow rate measuring means such as flow meter 21 measures the actual gas flow and supplies this information to the electronic circuit. Pressure measuring device 22 such at a well-known pressure transducer is positioned in gas line 33 downstream from the flow meter for supplying information to the electronic circuit. Gas passes pressure transducer 22 via gas line 33 to insufflation needle 14 for delivery into the body cavity of the patient.

In use, electronic circuit 12 receives information from pressure measuring means 17 and 22, flow rate measuring means 21, electronically controlled valve 20, and patient monitoring system 13. The information is preferably displayed on well-known display panel 23. When electronic circuit 12 is a microprocessor, for example, information also can be supplied by a surgeon or technician via the display panel. Electronic circuit 12 responds to the information provided, as suggested above, and controls the gas flow via the appropriate regulators and valves.

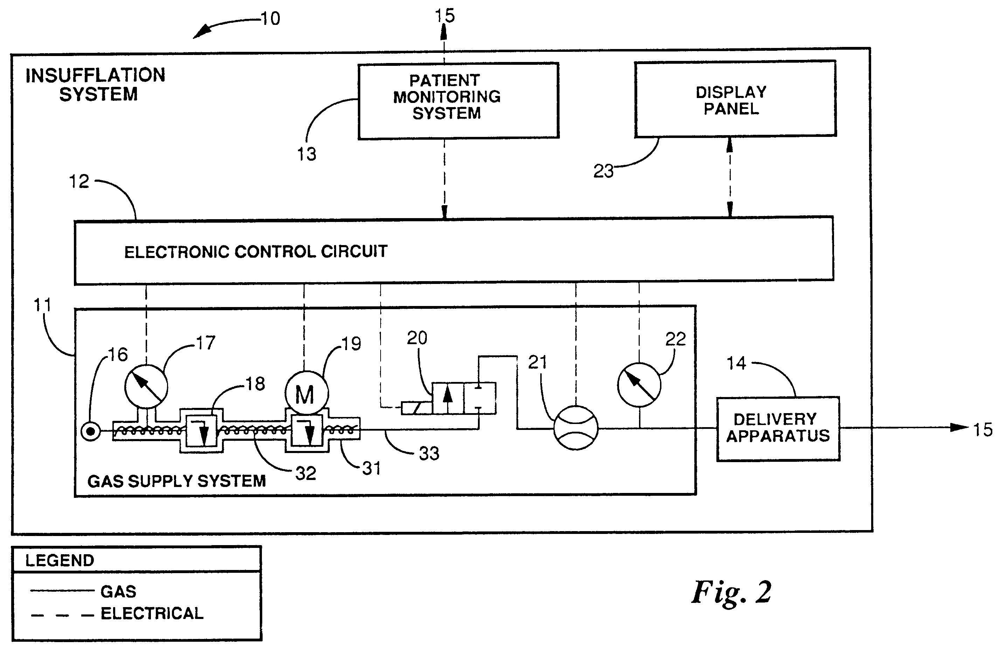

FIG. 2 depicts a schematic block diagram of an enhancement to the insufflation system of FIG. 1 with thermal insulation 31 and heating elements 32. Thermal insulation 31 partially surrounds pressure reducing means 18, regulation means 19, and gas line 33 extending therebetween. Thermal insulation 31 reduces the condensation of the pressurized gas passing through gas line 33 from gas storage reservoir 16. Heating elements 32 are positioned about gas line 33 proximate pressure measuring means 17, pressure reducing means 18, and regulation means 19 for warning the gas and greatly reducing the cooling effect off the pressurized gas. Thermal insulation 31 preferably surrounds heating elements 32 extending along the gas line.

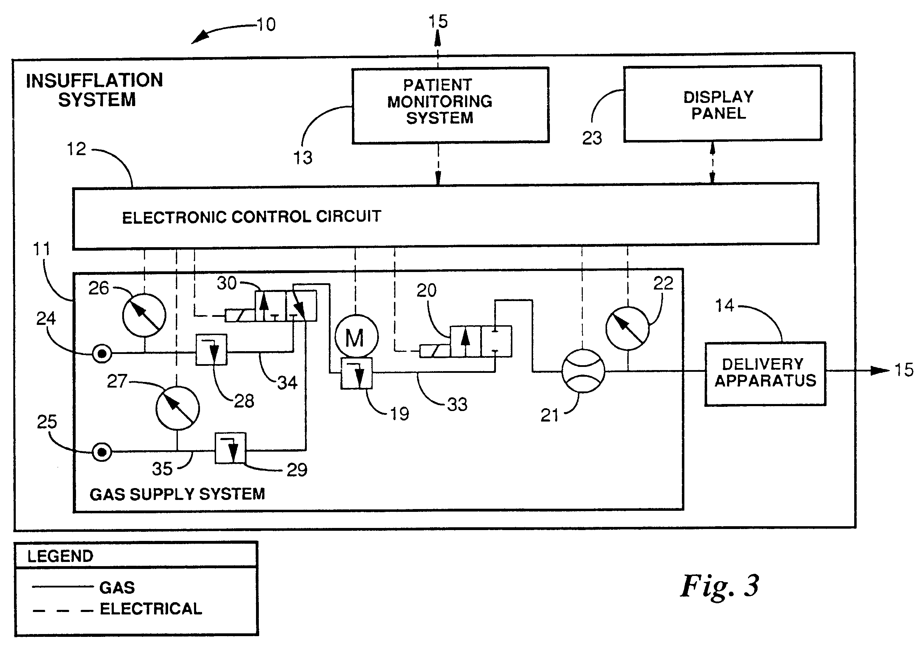

FIG. 3 depicts a schematic block diagram of another aspect of the insufflation system of the present invention with a gas supply system that accommodates two gas reservoirs. In this manner, a large volume of pressurized gas is available to the surgeon without the previously suggested disadvantages of using a single, relatively large pressurized gas storage reservoir. Standard sized, pressurized gas storage reservoirs 24 and 25 are connected via separate gas lines 34 and 35, respectively, to main gas line 33. Gas storage reservoirs 24 and 25 are also connected to separate pressure reducers 28 and 29, respectively. The pressure reducers reduce the high pressure in the gas storage reservoirs down to, for example, 40-50 kPa. The pressure reducers can incorporate into their construction a gas flow reducer and a safety valve. Separate measuring means 26 and 27 are positioned in the separate gas lines 34 and 35 between each gas storage reservoir and each pressure reducer for providing information to electronic circuit 12. Alternatively, the pressure measuring means is incorporated into the pressure reducer. Preferably, gas passes via gas line 33 to the insufflation needle from only one gas storage reservoir at a time. Electronically operated valve 30 provides for selecting which gas storage reservoir provides pressurized gas to the gas supply system.

It is to be understood that the above-described insufflation system is merely an illustrative embodiment of the principles of this invention and that other insufflation systems may be devised by those skilled in the art without departing from the scope of this invention as defined in the appended claims. For example, the insufflation system is contemplated to include more than two separate gas storage reservoirs.

## Claims

1. A gas insufflation system comprising a gas line (33) for delivery of insufflation gas when connected to a patient, an electronic control circuit (12) responsive to gas pressure and gas flow rate in the gas line, a gas supply system (11) coupled to and under control of the electronic control circuit for supplying the insufflation gas to the gas line, and a patient monitoring system (13) connected to said electronic control circuit (12) characterised in that said patient monitoring system (13) provides information representative of the gas absorption levels by a patient and/or information representative of cardiovascular complications or irregularities and in that the control circuit is responsive to the said information for reducing or stopping the delivery of gas to the patient.

2. The system according to claim 1, wherein a display panel (23) coupled to the electronic control circuit (12) is provided to display either or both of the said representative information.

3. The insufflation system of claim 1, wherein the gas supply system includes a first pressurized gas storage reservoir (16) a pressure measuring transducer (17) coupled to said electronic control circuit (12), a gas flow rate measuring trasducer (21) coupled to said electronic control circuit, and a gas regulator(19 or 20) all connected to said gas line.

4. The insufflation system of claim 3, wherein the gas supply system further includes an electronically operated valve (20) connected to said gas line and responsive to said electronic control system.

5. The insufflation system of claim 3, further comprising a second gas reservoir (25), a second pressure measuring transducer (27) coupled to said electronic control circuit (12), and an electronically controlled valve (30) coupled to said first and second gas reservoirs (16,25) and responsive to said electronic control circuit (12) for selectively connecting said gas line (33) to said first and/or second gas reservoirs (16,25).

6. The insufflation system of claim 3, wherein said gas supply system further comprises a pressure reducer (18) connected to said gas line (33) and said first gas reservoir (16)

7. The insufflation system of claim 6, wherein the gas supply system further comprises a heating element (32) disposed proximate said gas line (33) between said first gas reservoir (16) and at least one of said pressure reducer (18) and said gas regulator (19).

8. The insufflation system of claim 6, wherein said gas supply system further comprises thermal insulation (31) disposed proximate at least one of said pressure reducer (18) and said gas regulator (19).

9. The insufflation system of any preceding claim, further comprising a second pressure measuring transducer (22) coupled to said electronic control circuit (12) and said gas line (33) and connected to said gas line after said gas flow measuring transducer (21).

## Patentansprüche

1. Gasinsufflationssystem mit einer Gasleitung (33) zur Zuführung von Insufflationsgas bei Verbindung mit einem Patienten, einer auf Gasdruck und Gasströmungsgeschwindigkeit in der Gasleitung reagierenden elektronischen Steuerschaltung (12), einem Gaszufuhrsystem (11), das an die elektronische Steuerschaltung gekoppelt ist und unter deren Steuerung steht, um der Gasleitung das Insufflationsgas zuzuführen, und einem mit der elektronischen Steuerschaltung (12) verbundenen Patientenüberwachungssystem (13), dadurch gekennzeichnet, daß das Patientenüberwachungssystem (13) eine die Gasabsorptionsrate eines Patienten darstellende Information und/oder eine kardiovaskuläre Komplikationen oder Unregelmäßigkeiten darstellende Information liefert und daß die Steuerschaltung auf die Informationen dahingehend reagiert, die Gaszufuhr zu dem Patienten zu verringern oder zu stoppen.

2. System nach Anspruch 1, bei dem ein an die elektronische Steuerschaltung (12) gekoppeltes Anzeigefeld (23) zur Anzeige einer der beiden oder beider repräsentativen Informationen vorgesehen ist.

3. Insufflationssystem nach Anspruch 1, bei dem das Gaszufuhrsystem einen ersten Druckgasspeicher (16), einen an die elektronische Steuerschaltung (12) gekoppelten Meßwandler (17), einen an die elektronische Steuerschaltung gekoppelten Gasströmungsgeschwindigkeitsmeßwandler (21) und einen Gasregler (19 oder 20) enthält, die alle mit der Gasleitung verbunden sind.

4. Insufflationssystem nach Anspruch 3, bei dem das Gaszufuhrsystem weiterhin ein elektronisch betätigtes Ventil (20) enthält, das mit der Gasleitung verbunden ist und auf das elektronische Steuersystem reagiert.

5. Insufflationssystem nach Anspruch 3, weiterhin mit einem zweiten Gasspeicher (25), einem zweiten Druckmeßwandler (27), der an die elektronische Steuerschaltung (12) gekoppelt ist, und einem elektronisch gesteuerten Ventil (30), das an den ersten und den zweiten Gasspeicher (16, 25) gekoppelt ist und auf die elektronische Steuerschaltung (12) zur gezielten Verbindung der Gasleitung (33) mit dem ersten und/oder zweiten Gasspeicher (16, 25) reagiert.

6. Insufflationssystem nach Anspruch 3, bei dem das Gaszufuhrsystem weiterhin einen Druckminderer (18) umfaßt, der mit der Gasleitung (33) und dem ersten Gasspeicher (16) verbunden ist.

7. Insufflationssystem nach Anspruch 6, bei dem das Gaszufuhrsystem weiterhin ein Heizelement (32) umfaßt, das nahe der Gasleitung (33) zwischen dem ersten Gasspeicher (16) und dem Druckminderer (18) und/oder Gasregler (19) angeordnet ist.

8. Insufflationssystem nach Anspruch 6, bei dem das Gaszufuhrsystem weiterhin eine thermische Isolierung (31) umfaßt, die nahe dem Druckminderer (18) und/oder Gasregler (19) angeordnet ist.

9. Insufflationssystem nach einem der vorhergehenden Ansprüche, weiterhin mit einem zweiten Druckmeßwandler (22), der an die elektronische Steuerschaltung (12) und die Gasleitung (33) gekoppelt und hinter dem Gasströmungsmeßwandler (21) mit der Gasleitung verbunden ist.

## Revendications

1. Système d'insufflation de gaz comprenant une ligne de gaz (33) pour délivrer du gaz d'insufflation lorsqu'elle est connectée à un patient, un circuit de commande électronique (12) sensible à la pression de gaz et au débit de gaz dans la ligne de gaz, un système d'alimentation de gaz (11) couplé à et commandé par le circuit de commande électronique pour alimenter la ligne de gaz en gaz d'insufflation, et un système de contrôle du patient (13) connecté audit circuit de commande électronique (12), caractérisé en ce que ledit système de contrôle du patient (13) fournit des informations représentatives des niveaux d'absorption de gaz par un patient et/ou des informations représentatives de complications ou d'irrégularités cardio-vasculaires et en ce que le circuit de commande réagit auxdites informations en réduisant ou en arrêtant l'apport de gaz au patient.

2. Système selon la revendication 1, dans lequel un panneau d'affichage (23) couplé au circuit de commande électronique (12) est prévu pour afficher l'une ou l'autre ou les deux desdites informations représentatives.

3. Système d'insufflation selon la revendication 1, dans lequel le système d'alimentation de gaz comporte un premier réservoir de stockage de gaz sous pression (16), un transducteur (17) de mesure de pression couplé audit circuit de commande électronique (12), un transducteur (21) de mesure du débit de gaz couplé audit circuit de commande électronique, et un régulateur de gaz (19 ou 20), tous connectés à ladite ligne de gaz.

4. Système d'insufflation selon la revendication 3, dans lequel le système d'alimentation de gaz comporte en outre une soupape à commande électronique (20) connectée à ladite ligne de gaz et réagissant audit système de commande électronique.

5. Système d'insufflation selon la revendication 3, comprenant en outre un deuxième réservoir de gaz (25), un deuxième transducteur (27) de mesure de pression couplé audit circuit de commande électronique (12) et une soupape à commande électronique (30) couplée auxdits premier et deuxième réservoirs de gaz (16, 25) et réagissant audit circuit de commande électronique (12) pour connecter de manière sélective ladite ligne de gaz (33) auxdits premier et/ou deuxième réservoirs de gaz (16, 25).

6. Système d'insufflation selon la revendication 3, dans lequel ledit système d'alimentation de gaz comprend en outre un réducteur de pression (18) connecté à ladite ligne de gaz (33) et audit premier réservoir de gaz (16).

7. Système d'insufflation selon la revendication 6, dans lequel le système d'alimentation de gaz comprend en outre un élément chauffant (32) disposé à proximité de ladite ligne de gaz (33) entre ledit premier réservoir de gaz (16) et au moins l'un dudit réducteur de pression (18) et dudit régulateur de gaz (19).

8. Système d'insufflation selon la revendication 6, dans lequel ledit système d'alimentation de gaz comprend en outre une isolation thermique (31) disposée à proximité d'au moins l'un dudit réducteur de pression (18) et dudit régulateur de gaz (19).

9. Système d'insufflation selon l'une quelconque des revendications précédentes, comprenant en outre un deuxième transducteur (22) de mesure de pression couplé audit circuit de commande électronique (12) et à ladite ligne de gaz (33) et connecté à ladite ligne de gaz après ledit transducteur (21) de mesure du débit de gaz.
